# EUROPEAN PATENT APPLICATION

(11) **EP 3 900 720 A1**
(43) Date of publication of application: **27.10.2021**
(21) Application number: 21000108.7
(22) Date of filing: 20.04.2021
(51) Int. Cl.: A61K 31/352, A61P 3/00

(54) **COMPOSITION BASED ON GENISTEIN FOR THE TREATMENT OF OBESITY**

(30) Priority: 22.04.2020 IT 202000008554
(71) Applicant: Sunnutrapharma S.r.l., 98125 Messina (IT)
(72) Inventor: Squadrito, Francesco, I-98125 Messina (IT); Bitto, Alessandra, I-98125 Messina (IT)
(74) Representative: Garavelli, Paolo

(57) **Abstract**

A composition based on genistein is described, for use in inducing a switch of adipose cells from white to brown, thus promoting lipid metabolism in obesity and/or metabolic syndrome.

## Description

The present invention refers to a new therapeutic use of genistein, for the treatment of obesity and/or metabolic syndrome.

Obesity and metabolic syndrome are responsible for most cardio-cerebrovascular diseases: specific drugs for obesity are few and do not have a specific action on adipose tissue.

These drugs are orlistat, liraglutide, and the naltrexone/bupropion combination. The first reduces the absorption of fats in the gastrointestinal tract, while liraglutide causes satiety and stimulates the production of insulin causing a reduction in blood sugar. Finally, the naltrexone/bupropion combination (used individually in opiate and alcohol addictions and in nicotine addictions) reduces appetite and stimulates the production of endorphins which act on the nerve pathways causing a powerful anorectic effect. However, these drugs do not reduce the number of fat cells or modify the distribution of body fat. Furthermore, these therapies require careful patient monitoring to avoid the onset of serious adverse reactions.

In Italy prevalence and incidence of obesity are constantly growing. ISTAT data show that about 10% of the population is obese and 35% is overweight. The resulting health costs are equal to 9% of the national health system budget, reducing GDP by 2.8% and weighing on each citizen for 289 Euros in additional taxes per year. In addition, the effects that obesity and overweight have on health cause a reduction in the average life span of Italians affected by this pathology of 2.7 years compared to normal weight.

Finally, at working level, production capacity is significantly reduced, causing a loss of 571,000 full-time workers per year. Although our country has launched awareness campaigns, issued guidelines to enhance proper physical activity and an optimal healthy diet, and lastly required all producers to label the nutritional values of foods, the problem remains difficult today to stem, making pharmacological intervention frequently necessary.

The present invention is based on the recognition of the fact that the use of genistein is able to modify the characteristics of fat cells. In fact, fat cells exist in at least two "forms", white and brown. White fat cells are responsible for the accumulation of lipids and determine the production of pro-inflammatory molecules called adipokines. Brown cells, on the other hand, are rich in mitochondria and use lipids to create heat, through oxidation in the mitochondria. These two cell types were thought to be in a static condition, however it has been discovered that it is possible that there is a trans-differentiation from white to brown cells (which are called beige in this case) if subjected to adequate stimuli. The main stimuli are for example cold and some molecules that are re-leased when exposed to low temperatures, through the action on beta3-adrenergic receptors that are also found on fat cells and stimulate thermogenesis, the use of lipids and production of new mitochondria. This process is not easy to stimulate and often takes some time. At the moment a trans-differentiation of adipocytes has been obtained only in the ways described above.

In previous studies it was shown that genistein had an activity that is expressed both through the estrogen receptor and through the nuclear PPAR-gamma receptor. It was also previously observed that genistein has some efficacy in reducing plasma lipid levels both in experimental animals and in subjects with metabolic syndrome.

The invention intends to cover both the use of genistein alone and in combination with other compounds (vitamins, hydroxyl-citric acid, lycopene and other flavonoids) at any dosage in the range of 0-1000 mg; preferably, the dosage of genistein alone or in association with other compounds is in the range from 10 to 100 mg. A study on the dosages is described in patent application US2015190365; the range of doses reported shows that in particular doses comparable to 10-100 mg (for human use) were derived from studies on the plasma concentration of genistein in subjects of Asian origin with soy-based diets.

Genistein is an isoflavone found in particular in soy and other legumes, but also vegetables such as parsley are particularly rich in it. It is important not to confuse genistein with genistin which is its glycosylated form (i.e. with a sugar molecule linked to the structure) which is more difficult to absorb in the intestinal mucosa and with reduced pharmacological properties. Genistein can be of an extractive or synthetic nature (often called bonistein), of the extractive one, however, formulations are available that have up to 98% purity.

The objects and advantages of the invention, as will emerge from the following description, are achieved with a genistein-based composition for the treatment of obesity such as that described in the main claim. Preferred embodiments and non-trivial variants of the present invention form the subject of the dependent claims.

It is understood that all attached claims form an integral part of the present description.

It will be immediately obvious that innumerable variations and modifications (for example relating to shape, dimensions, arrangements and parts with equivalent functionality) can be made to what is described without departing from the scope of the invention as appears from the attached claims.

As part of the invention, the activity of genistein in stimulating the trans-differentiation from white to brown adipocytes was demonstrated through the tests carried out and reported in the graphs of the attached drawings, in which:
- Figure 1 shows an image of the 3T3-L1 control cells after differentiation into white adipocytes (A), as highlighted by the arrows indicating cells with large lipid droplets inside them; and of the same cells after 24 hours of exposure to genistein at doses of 50 µM (B) and 100 µM (C), with the arrowheads indicating the presence of small lipid droplets inside them; the graph representing the reduction in the accumulation of triglycerides caused by Genistein, relating to experiment 1;
- Figure 2 shows the graphs relating to experiment 1 which represent the ability of genistein to induce trans-differentiation;
- Figure 3 shows the graphs relating to experiment 2 which represent the fact that the effect of genistein does not depend on the beta estrogen receptor (ERbeta);
- Figure 4 shows the graphs relating to experiment 3 which represent the fact that the effect of genistein depends on the PPAR-gamma receptor; and
- Figure 5 shows the graphs relating to experiment 4 which represent the fact that the combined block of the two receptors (PPARgamma and ERbeta) with the antagonists GW9662 and PHTP cancels the effect of genistein.

### Experiment 1:

In the first experiment, 3T3-L1 cells were used, which are pre-adipocytes and were differentiated into white adipocytes using a standard protocol that involves the use of culture media supplemented with IBMX, dexamethasone and insulin. At the end of the differentiation the cells were observed with a staining (Oil red-O) that allows to highlight the lipids and the typical large lipid droplets inside the cells were noted. To evaluate the ability of genistein to induce trans-differentiation, different doses of genistein were added to the cultured cells (from 10 to 200 micro-molar) and after 24 hours the staining with oil red-O was repeated which showed a reduction in the accumulation of lipids and the appearance of cells containing small lipid droplets, this phenotype is typical of brown adipocytes.

Furthermore, by analysing the cells thus stained with a spectrophotometer, the percentage reduction in the accumulation of lipids was evaluated. The control cells were assumed to have a quantity of lipids equal to 100% and using a proportion the percentage of lipids present with the various doses of genistein was evaluated, these concentrations were significantly reduced in just 24 hours of treatment.

These cells have also been used for the extraction of RNA and the evaluation of the expression of genes involved in the trans-differentiation process, such as UCP-1, PRDM-16, PPARalpha, PPARgamma and DIO2. All of these were increased in genistein-treated cells compared to untreated cells already differentiated into white adipocytes.

### Experiment 2:

In the second experiment it was investigated whether this trans-differentiation process was dependent on the activity of genistein through the beta-type estrogen receptor (which should be the most stimulated at the doses used). A receptor inhibitor, PHTPP, was then used together with genistein, and both substances were left to act for 24 hours in cells already differentiated into white adipocytes. At this point, the extraction of the RNA and the evaluation of the trans-differentiation genes were repeated. Surprisingly it was observed that the reduction in expression of target genes, when cells were co-treated with PHTPP and genistein, was actually modest, suggesting that the mechanism of action was not (at least not entirely) dependent on the receptor. beta-type estrogenic.

### Experiment 3:

In this experiment, considering that genistein can also act through stimulation of the PPARgamma receptor and that this receptor seems to have a stimulating effect of trans-differentiation, an inhibitor of this receptor, GW9662, was used, always in co-administration with genistein to 24 hours at the end of the cell differentiation period into white adipocytes. RNA was re-extracted and the target genes evaluated and their expression reduced to levels observed in control cells. This result was completely unexpected, precisely because the beta estrogen receptor seemed to have, according to the literature, a greater ability to exert the positive effects of genistein.

### Experiment 4:

To eliminate the doubt that other receptors (such as the estrogen alpha receptor or PPARalpha) which have also been indicated as receptors to which genistein can bind were involved in the trans-differentiation process, the experiment was repeated by subjecting the cells (always for 24 hours) to treatment with genistein and with both compounds (GW9662 and PHTPP). RNA was then extracted and the target genes evaluated by observing a complete reduction of their expression, up to cancellation, as in the case of UCP-1 which is called Uncoupling-1 and is the main gene involved in the mitochondria activity of brown adipocytes.

In conclusion, the data observed in the experiments indicate that:
- genistein can transform white fat cells into brown ones (while other published experiments use genistein in the differentiation process, in the experiments carried out by the inventors it was used after the cells had a clear "white" phenotype);
- the mechanism of action is strictly dependent on the increase (up to 15 times) of the expression of the gene for UCP-1;
- this process is strictly dependent on the presence of the PPARgamma receptor and not on the estrogen receptor, therefore male subjects or women in menopause (in both cases there are few estrogen receptors) can benefit from treatment with genistein;
- trans-differentiation is inducible in a rapid time (in cells 24 hours).

## Claims

1. Composition based on genistein for use in inducing a switch of adipose cells from white to brown, thus promoting lipid metabolism in obesity and/or metabolic syndrome.

2. Composition for use according to claim 1 for inducing lipid metabolism in obesity and/or metabolic syndrome through oral administration.

3. Composition for use according to claim 1 or 2, wherein said composition comprises genistein administered at any dosage ranging from 0 to 1000 mg.

4. Composition for use according to claim 1 or 2, wherein said composition comprises genistein in association with other compounds administered at any dosage ranging from 0 to 1000 mg.

5. Composition for use according to any of the preceding claims, in which the genistein is of an extractive nature.

6. Composition for use according to claim 5, wherein the genistein is in a formulation having up to 98% purity.

7. Composition for use according to any one of claims 1 to 4, in which the genistein is synthetic in nature.

8. Composition for use according to any one of the preceding claims, comprising a pharmaceutically acceptable vehicle.

9. Composition for use according to any one of the preceding claims in the form of tablets, capsules, pills, granules, soft tablets, oral gels for oral administration.

10. Use of the composition according to any of the preceding claims for inducing lipid metabolism to treat obesity and/or metabolic syndrome in a human subject.
